(19) Europäisches Patentamt / European Patent Office / Office européen des brevets

(11) **EP 3 315 075 B1**

(12) **EUROPEAN PATENT SPECIFICATION**

(45) Date of publication and mention
of the grant of the patent:
**10.07.2019   Bulletin 2019/28**

(51) Int Cl.:
*A61B 8/08* (2006.01)          *A61B 5/05* (2006.01)

(21) Application number: **16196101.6**

(22) Date of filing: **27.10.2016**

(54) **SYSTEM AND METHOD FOR COMBINED MICROWAVE AND ULTRASOUND IMAGING**

SYSTEM UND VERFAHREN ZUR KOMBINIERTEN BILDGEBUNG MIT HILFE VON MIKROWELLEN
UND ULTRASCHALL

SYSTÈME COMBINÉ D'IMAGERIE MICRO-ONDE ET ULTRASONORE ET PROCÉDÉ ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**02.05.2018   Bulletin 2018/18**

(73) Proprietor: **Micrima Limited
Bristol BS2 0EL (GB)**

(72) Inventors:
• **BANNISTER, Peter
  Bristol
  BS20 0EL (GB)**
• **BORE, Chris
  Bristol
  BS2 0EL (GB)**

(74) Representative: **Haseltine Lake Kempner LLP
Redcliff Quay
120 Redcliff Street
Bristol BS1 6HU (GB)**

(56) References cited:
WO-A1-2012/048020          WO-A1-2014/039017
CN-A- 105 708 492          CN-B- 102 499 713
US-A1- 2007 276 240        US-A1- 2010 036 240

• **PREECE ALAN W ET AL: "MARIA M4: clinical
evaluation of a prototype ultrawideband radar
scanner for breast cancer detection", JOURNAL
OF MEDICAL IMAGING, vol. 3, no. 3, 1 July 2016
(2016-07-01), pages 33502-1-33502-7,
XP060075332, ISSN: 2329-4302, DOI:
10.1117/1.JMI.3.3.033502 [retrieved on
2016-07-20]**

## Description

The invention relates to a medical imaging system and method.

Various medical imaging techniques are known for examining the human body. Such imaging techniques may be used to interrogate tissues and organs in order to identify abnormalities, such as tumours or lesions. For example, X-ray (mammography), microwave imaging, ultrasound, MRI are all common imaging modalities. Such techniques are commonly used to examine breast tissue, but may also be used in other areas of the body, such as the liver, pancreas, prostate, thyroid, lungs, ovaries and lymph nodes.

The quality of the image produced by an imaging modality is a key factor in its uptake. In particular, the spatial resolution and presence or absence of artefacts in the image are fundamental properties which may be considered when selecting an imaging modality. As a result, there is generally a desire to improve the content of the image produced by a medical imaging system.

CN 102499713 B discloses a medical imaging system, having a microwave antenna configured to emit microwaves towards an object such that an ultrasonic wave is generated and detected by a transducer to image the object. The microwave antenna and ultrasonic transducers are arranged relative to a hemispherical support.

CN 105708492 A discloses a system comprising a B-ultrasound imaging unit for generating a B-mode image, a microwave imaging unit for generating a microwave image, and a fusion processing unit for image registration. The microwave transceiver antenna units are arranged on an hemispherical support.

The invention seeks to provide a system which improves the quality of the image obtained using a microwave antenna array.

In accordance with an aspect of the invention, there is provided a medical imaging system comprising: a microwave antenna array comprising a plurality of microwave antennae which are spaced from one another; an ultrasonic transducer array comprising a plurality of ultrasonic transducers which are spaced from one another and interspersed between the microwave antennae; wherein the plurality of microwave antennae define a transmitting antenna configured to transmit microwave signals so as to illuminate a body part of a patient and define receiving antennae configured to receive the microwave signals following interaction with the body part; and wherein the plurality of ultrasonic transducers define a transmitting transducer configured to transmit ultrasonic waves so as to insonate the body part of the patient and define receiving transducers configured to receive the ultrasonic waves following interaction with the body part; the system further comprising: a processor configured to process the received microwave signals and the received ultrasonic waves and generate an image of the internal structure of the body part. The microwave antennae and ultrasonic transducers are formed on a hemi-

spherical substrate which is contoured or contourable to conform to the body part.

The microwave antennae and the ultrasonic transducers may be wavelength-matched so as to allow similar data capture, processing, and rendering to be performed for the microwave and ultrasound modalities. The wavelength of the ultrasonic waves and the wavelength of the microwave signals may be substantially equal.

The wavelength of the ultrasonic waves and the wavelength of the microwave signals may differ by up to 10%.

The microwave antennae and ultrasonic transducers may emit a continuous-wave signal which is reconstructed using a phased array or delay-and-sum algorithm.

The processor may be configured to generate a microwave image based on the received microwave signals and an ultrasound image based on the received ultrasonic waves, and the microwave and ultrasound images may be spatially registered.

The microwave image and ultrasound image may be overlaid on one another to form a compound image.

The microwave antennae and the ultrasonic transducers may be arranged in corresponding formations and the microwave antenna array and the ultrasonic transducer array may be offset from one another such that the ultrasonic transducers are interspersed between the microwave antennae.

The microwave antenna and ultrasonic transducer arrays may have a plurality of configurations defining positions of the plurality of microwave antennae and ultrasonic transducers relative to the body part, wherein in each configuration the plurality of microwave antennae are located at positions which are unoccupied by microwave antennae in the other configurations and the plurality of ultrasonic transducers are located at positions which are unoccupied by ultrasonic transducers in the other configurations. The system may further comprise: an actuator configured to move the microwave antenna array and ultrasonic transducer array between the plurality of configurations so as to position the plurality of microwave antennae and the plurality of ultrasonic transducers at previously unoccupied positions. The processor may be configured to obtain a data set for the microwave signals and ultrasonic signals produced in each of the plurality of configurations and to generate an image of the internal structure of the body part from a concatenation of the data sets.

The physical spacing between the microwave antennae may be greater than half the wavelength of the microwave signal and an effective spacing between the microwave antennae at the positions defined by the plurality of configurations may be less than half the wavelength of the microwave signal and/or the physical spacing between the ultrasonic transducers may be greater than half the wavelength of the ultrasonic signal and an effective spacing between the ultrasonic transducers at the positions defined by the plurality of configurations may be less than half the wavelength of the ultrasonic signal.

The microwave antennae and ultrasonic transducers may each be positioned in a formation formed from a regular tessellation of a polygon which is tilted with respect to a direction of movement of the antennae.

The microwave antennae and ultrasonic transducers may be positioned in a formation which is formed from an inverse Mercator projection of the regular tessellation.

The polygon may be a square.

The formation may be tilted at an angle which is between 20 degrees and 35 degrees.

The formation may be tilted at an angle of 27 degrees.

In accordance with an aspect of the invention, there is provided a medical imaging method comprising: illuminating a body part of a patient with microwave signals emitted by a transmitting antenna of a microwave antenna array; receiving the microwave signals following interaction with the body part at a plurality of receiving antennae of the microwave antenna array to obtain a data set; insonating the body part with ultrasonic waves emitted by a transmitting transducer of an ultrasonic transducer array; receiving the ultrasonic waves following interaction with the body part at a plurality of receiving transducers of the ultrasonic transducer array to obtain a data set; and processing the received microwave signals and the received ultrasonic waves to generate an image of the internal structure of the body part. The microwave antennae and ultrasonic transducers are formed on a hemispherical substrate and the substrate is contoured or contourable to conform to the body part.

The microwave antennae and the ultrasonic transducers may be wavelength-matched so as to allow similar data capture, processing, and rendering to be performed for the microwave and ultrasound modalities.

A microwave image based on the received microwave signals and an ultrasound image based on the received ultrasonic waves may be generated, and the microwave and ultrasound images may be spatially registered.

The microwave image and ultrasound image may be overlaid on one another to form a compound image.

For a better understanding of the invention, and to show more clearly how it may be carried into effect, reference will now be made, by way of example, to the accompanying drawings, in which:

Figure 1 is a system diagram of a medical imaging system according to an embodiment of the invention;

Figure 2 is a schematic view of the medical imaging system;

Figure 3 is a flowchart depicting a sampling method;

Figure 4 is a plan view of a transceiver array of the medical imaging system in a first configuration;

Figure 5 is a plan view of the transceiver array in a second configuration;

Figure 6 is a plan view of the transceiver array in a third configuration;

Figure 7 shows the positions of the transceivers for each configuration shown in Figures 4 to 6 overlaid on one another; and

Figure 8 is an illustrative example showing a translation of a tilted tessellation.

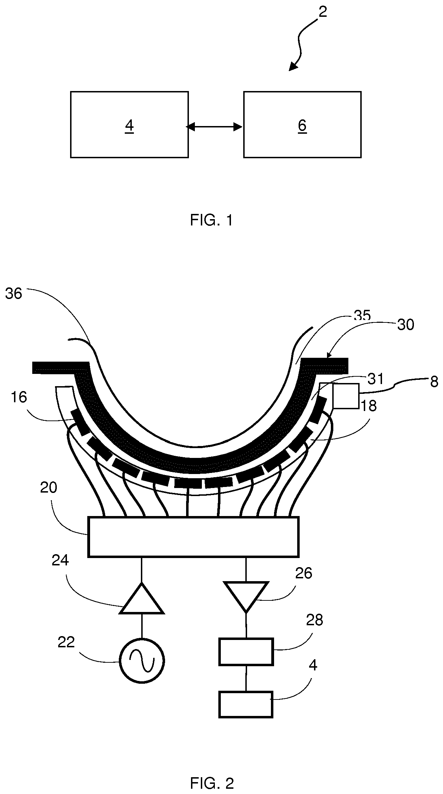

Figure 1 shows a medical imaging system 2 according to an embodiment of the invention. The medical imaging system generally comprises a processor 4, a transceiver array 6 in communication with the processor 4.

As shown in Figure 2, the transceiver array 6 comprises a plurality N of transceivers 16 which are arranged over the surface of, or within, a shell substrate 18. The shell 18 has a curved profile as shown such that the transceivers 16 form a 3D array. In particular, the shell 18 is hemi-spherical and is configured to approximate the shape of a breast. The transceivers 16 are arranged over the shell 18 such that they all point to a common focal point.

The transceivers 16 comprise a plurality of ultrasonic transducers and a plurality of microwave antennae (not separately identified in Figure 1). The ultrasonic transducers form a first array and the microwave antennae form a second array. The ultrasonic transducers and the microwave antennae are arranged in identical or similar formations or patterns, with the two arrays offset from one another such that the ultrasonic transducers and the microwave antennae are interspersed with one another.

The transceivers 16 are each electrically connected to a switching matrix 20. The switching matrix 20 is in turn connected to both a transmit path and a receive path. The transmit path comprises a signal generator 22 coupled to an amplifier 24. The receive path comprises an amplifier 26 coupled to a detector 28 and the processor 4.

The switching matrix 20 selectively couples each of the transceiver 16 to either the transmit path or the receive path.

In practice, the ultrasonic transducers and the microwave antennae may form entirely separate circuits. In particular, the ultrasonic transducers may have a dedicated ultrasonic signal generator and the microwave antennae may have a dedicated microwave signal generator; however, for simplicity these functions have been amalgamated into a single component.

The transceiver array 6 is operated in a multistatic fashion. Specifically, in a microwave capture phase, the switching matrix 20 is controlled so as to connect one of the microwave antennae 16 to the transmit path and the remaining microwave antennae 16 to the receive path. The signal generator 22 generates a stepped frequency continuous wave (CW) signal which is amplified by the amplifier 24 and then transmitted by the microwave antenna 16 connected to the transmit path. The stepped frequency continuous wave signal is a sequential series of pulses of continuous wave energy, where each pulse has its frequency stepped up across a range of frequencies, typically within the 3-8 GHz range. The other microwave antennae 16 receive the transmitted signal and the received signal is detected and then recorded by the processor 4.

Similarly, in an ultrasonic capture phase, the switching matrix 20 is controlled so as to connect one of the ultrasonic transducers 16 to the transmit path and the remaining ultrasonic transducers 16 to the receive path. The signal generator 22 generates an ultrasonic signal (which may again be a stepped frequency continuous wave (CW) signal) which is amplified by the amplifier 24 and then transmitted by the ultrasonic transducer 16 connected to the transmit path. The other ultrasonic transducers 16 receive the transmitted signal and the received signal is detected and then recorded by the processor 4.

The signals generated by the microwave antennae and the ultrasonic transducers are matched such that they have the same (or very similar) wavelengths.

The wavelength $L$ of waves is the ratio of the speed of propagation c to the frequency f:

$$L = c/f$$

For microwaves the speed of propagation in human tissue (specifically breast tissue) is 1e8 m/s, whereas for ultrasound the speed of propagation in human tissue is 1.54e3 m/s.

Thus the wavelength of ultrasound is 1.54e3/1e8 = 1.54e-5 times as long, for the same frequency, as that of microwaves. Therefore, to achieve the same wavelength of ultrasound as microwaves an ultrasound frequency that is (1/1.54e-5) times that of the microwaves is used. That is:

- 3 GHz microwave → 46.2 kHz ultrasound

- 5 GHz microwave → 77 kHz ultrasound

- 8 GHz microwave → 123.2 kHz ultrasound

Low-frequency ultrasound (which is outside of the range typically used for ultrasound imaging - usually in the range of 1-20 MHz) is therefore used to achieve this matching.

As shown in Figure 2, the shell 18 receives a cup 30. The cup 30 has a complementary shape to the shell 18 such that if fits snugly within the shell 18. A layer of coupling fluid (dielectric constant controlled fluid) may be inserted in the gap 31 between the shell 18 and the cup 30 so as to improve the coupling between the transceivers 16 and the cup 30 in order to minimise signal loss and thus improve transmission of the microwave and ultrasonic signals.

The microwave antennae 16 may be as described in WO 2009/060181. In particular, the microwave antennae 16 may comprise a slot formed in a conductive element, the slot having a rectangular external boundary defined by a substantially closed internal edge of the conductive element. A microstrip feed line may be spaced from the conductive element by a dielectric substrate with the distal end of the line positioned at the geometric centre of the slot.

The ultrasonic transducers may be any suitable transducer. In particular, the transducers may be a 400ETR080 transducer manufactured by Pro-Wave Electronics Corporation or similar. Such transducers have a diameter of less than 10mm and so can be easily interspersed between the microwave antennae. However, it will be appreciated that other transducers may be used.

As the ultrasonic transducers are interspersed between the microwave antennae, it is more difficult to achieve a high density of each type of transceiver. Accordingly, the system 2 may use an actuator (not shown) to rotate the transceiver array 6 so that the transceivers 16 assume positions which were previously unoccupied by the same type of transceiver. Although the positions of the transceivers 16 may overlap to a certain extent, the term "unoccupied" is used herein to refer to new sampling positions. The rotation of the transceiver array 6 therefore creates a "virtual" array which has a higher density of transceivers 16.

In use, a patient lies in a prone position such that their breast 36 sits in the cup 30. A layer of coupling fluid may also be provided in the gap 35 between the cup 30 and the breast 36 in order to improve coupling between the antennae 16 and the breast 36.

Although not shown, one or more inserts may be placed inside the cup 30 so as to enable a better fit between the internal surface of the cup 30 and the breast 36. For example, a plurality of such inserts may be provided, each having different shapes and sizes, to enable the system to be better adapted to breasts of different shapes and sizes. The inserts may be made from the same material as the cup (e.g. ceramic).

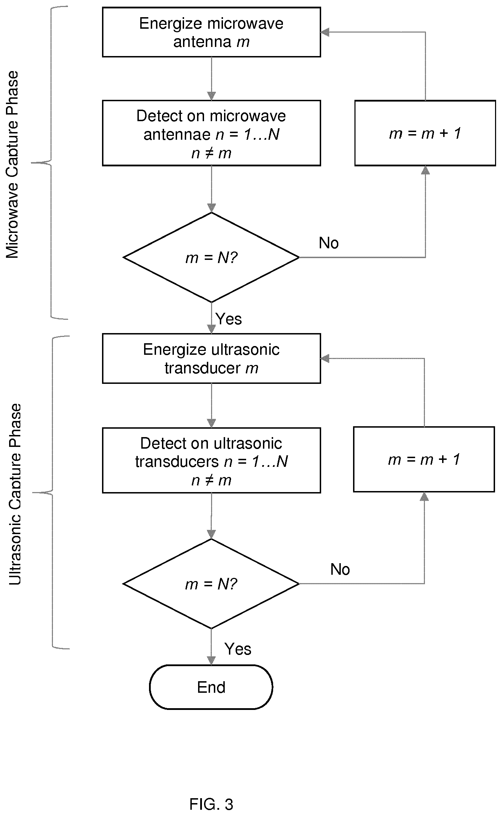

Figure 3 shows a flowchart of a data acquisition method. As shown, the data acquisition method comprises a microwave capture phase and an ultrasonic capture phase which are performed consecutively. In the microwave capture phase, the switching matrix 20 connects a microwave antenna $m$ of the $N$ microwave antennae to the transmit path. All other microwave antennae 16 ($n = 1...N, n \neq m$) are connected to the receive path. The microwave antenna 16 connected to the transmit path illuminates the breast 36 with the microwave signal. The signal is scattered by the breast tissue and the scattered signal is received at each of the non-transmitting antennae 16 where it is detected (possibly in a time-sharing arrangement) and recorded. If $m \neq N$, the switching matrix 20 steps to the next microwave antenna 16 ($m = m + 1$) to be connected to the transmit path. This is repeated until all microwave antennae 16 have been connected to the transmit path.

This process is then repeated for the ultrasonic transducers in the ultrasonic capture phase. The switching matrix 20 connects an ultrasonic transducer $m$ of the $N$ ultrasonic transducers to the transmit path. All other ultrasonic transducers 16 ($n = 1...N, n \neq m$) are connected to the receive path. The ultrasonic transducer 16 connected to the transmit path insonates the breast 36 with the ultrasonic signal. The signal is reflected, scattered and/or attenuated by the breast tissue and the signal is received at each of the non-transmitting ultrasonic transducers 16 where it is detected (possibly in a time-sharing arrangement) and recorded. If $m \neq N$, the switching matrix 20 steps to the next ultrasonic transducers 16 ($m = m + 1$) to be connected to the transmit path.

This is repeated until all ultrasonic transducers 16 have been connected to the transmit path.

It will be appreciated that the ultrasonic capture phase and microwave capture phase may be performed concurrently (possibly alternating between microwave and ultrasonic signals) if desired.

If additional data sets are required, then an actuator may be used to rotate the array 6 relative to the breast 36 while retaining the breast in position. The acquisition process may then be repeated with the antenna array 6 in the new configuration. Similarly, the array 6 may be rotated in-between the microwave capture phase and the ultrasound capture phase so that the ultrasonic transducers are located in the same positions as the microwave antennae.

The processor 4 may record the relative difference between the measured phase and amplitude of the transmitted signal as compared to the phase and amplitude of the received signal, recorded as a complex number (having real and imaginary parts).

The signal detected at each transceiver 16 is affected by scattering, reflection and/or attenuation arising from objects within the imaged volume (i.e. the breast 36). In particular, tumours can generate significant reflections as they exhibit much higher dielectric properties than adipose and connective tissues due to their significant water content and so can be identified in the acquired data.

The acquired data sets may be used by the processor 4 to construct an image of the internal structure of the breast 36. In particular, a microwave image may be obtained from the microwave data set acquired during the microwave capture phase and an ultrasound image may be obtained from the ultrasound data set acquired during the ultrasonic capture phase. The microwave and ultrasound images may be spatially registered so that a direct comparison can be made between the different tissue properties detected using the two imaging modalities. The data sets may also be combined by, for example, overlaying the microwave and ultrasound images on one another (or prior to image generation) to form a compound image which displays both sets of properties.

The data reconstruction may be performed using Phased Array (frequency domain), Delay and Sum (DAS - time domain) techniques or any other suitable technique, such as 3D Fourier Transformation, Back Projection, etc.

As described above, the rotation of the transceiver array 6 relative to the breast 36 during the image capture process moves the microwave antennae to positions which were previously unoccupied by microwave antennae and moves the ultrasonic transducers to positions which were previously unoccupied by ultrasonic transducers. The effective spacing of the microwave antennae and ultrasonic transducers in the virtual array is therefore smaller than the physical spacing of the microwave antennae and ultrasonic transducers on the shell 18. This may therefore improve the resolution of the images. The effective spacing of each type of transceiver 16 in the virtual array is preferably less than half the wavelength of the respective signal in order to prevent grating lobes from occurring in the image. Owing to the size of the transceivers 16 and the interspersion of microwave antennae and ultrasonic transducers, such spacing may not otherwise be physically achievable.

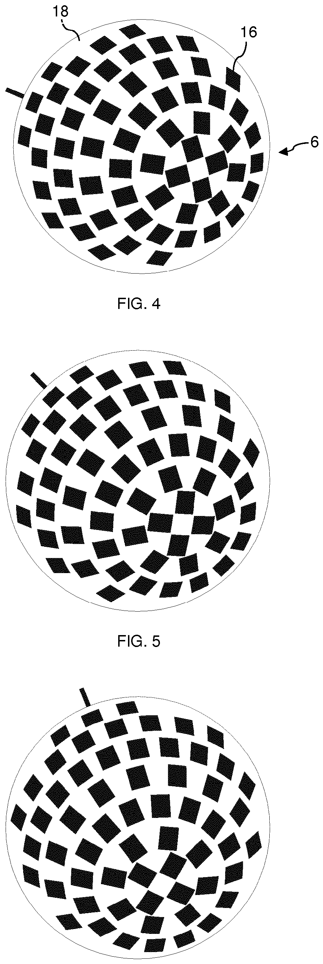

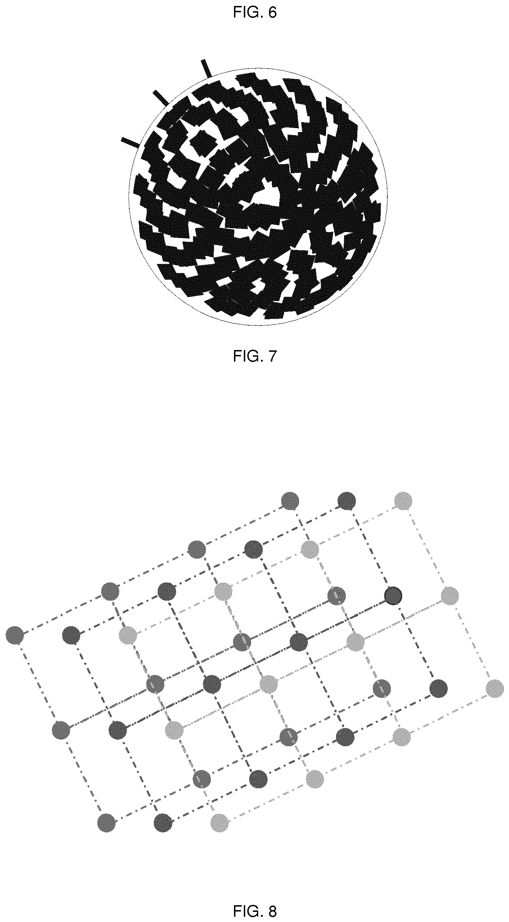

Although the transceivers 16 may be positioned over the shell 18 in any arrangement which causes them to be moved to previously unoccupied positions, Figures 4 to 6 show a preferred arrangement which maximises coverage. Figure 4 shows the transceiver array 6 in a first configuration and Figures 5 and 6 show the transceiver array 6 in second and third configurations where the array has been rotated about an axis of rotation which is preferably aligned with the axis of symmetry of the transceiver array 6. Figures 4 to 6 show only one type of transceivers from the microwave antennae and ultrasonic transducers. However, a corresponding arrangement would be used for the other type of transceivers which corresponds with but is offset from the first type of transceivers so that they are interspersed with one another.

The arrangement shown in Figures 4 to 6 is based on a projection into a hemispherical form of a tilted regular tessellation (tiling) of polygons, where the corners of the tiles define the positions of the transceivers 16.

For a simple tessellation of unit squares, a horizontal translation by 0.5 will generate a virtual array which has a reduced effective spacing in the horizontal direction, but which retains the same vertical spacing. In contrast, a diagonal translation (movement along two axes of motion - vertical and horizontal) will reduce the effective spacing in both the horizontal and vertical directions. This same effect can be achieved by instead tilting the tessellation of squares and then performing a translation along a single axis (e.g. horizontal), as shown in Figure 8.

By tilting the tessellation the corners of the squares move along paths which are all offset from one another, rather than moving along common paths. A uniform vertical spacing between the corners may be achieved by tilting the square tessellation by 26.565 degrees ($=\tan^{-1}(0.5)$). This maximises the vertical separation between the corners.

As described above, the arrangement shown in Figures 4 to 6 is a projection of the tilted tessellation into a hemispherical form. The tilted tessellation may be transformed into a hemispherical form using an inverse Mercator projection or other map projection. Rotation of the transceiver array 6 about its axis thus causes the transceivers 16 to occupy new positions which are spaced between the original positions of the transceivers 16 so as to define a virtual array which has a substantially uniform density with an effective spacing which satisfies the half wavelength criterion. Figure 7 shows the positions of the transceivers 16 for each configuration overlaid on one another and thus demonstrates the density of the virtual array.

Typically in a hemispherical array where the transceivers are separated by a single angular distance A, the angular rotation for each increment will be A/3, thereby increasing the virtual array density by a factor of three.

The density of the positions shown in Figure 7 is sufficient to produce a concatenated data set which is completely determined. In other words, from the concatenated data set, the interference pattern that is the scattered field may be calculated at any spatial position, including at positions other than those of the samples. Thus from a set of discrete samples complete information may be obtained about the field at all spatial positions. This avoids artefacts such as grating lobes from occurring within the image. The movement of the transceivers 16 reduces the effective spacing between the transceivers 16. This therefore allows larger and/or fewer transceivers 16 to be used and for microwave antennae and ultrasonic transducer sets to be interspersed with one another while still obtaining an effective spacing which is less than half the wavelength.

Although the transceiver array 6 has been described as having a plurality of discrete configurations/positions, the data may instead be acquired from a continuously moving array. Further, the entire transceiver array 6 need not rotate and the individual transceiver 16 may instead assume different positions within the shell 18 in each configuration of the transceiver array 6.

As described above, the signals generated by the microwave antennae and the ultrasonic transducers are matched such that they have the same (or similar) wavelengths. Under these conditions, the reconstruction for the ultrasound modality will be the same as, and therefore the image will be commensurate with, that for the microwave modality. In other words, the matched wavelengths ensures that the ultrasound and microwave imaging has the same scale and allows the same processing to be performed. In particular, a 3D continuous wave phased array reconstruction may be used for both imaging modalities. The data capture, processing, and rendering is therefore identical for both imaging modalities. The combined use of ultrasound and microwave energies provides enhanced detection of different internal materials of the body part, which improves the information content of the resulting images of the body part. In particular, the ultrasound and microwave imaging modalities are able to detect both differences in mechanical properties as well as differences in electromagnetic properties.

It will be appreciated that the wavelengths need not be identical. In particular, there is uncertainty regarding the background permittivity and thus the refractive index which leads to variation in the wavelength within the medium. This may be expected to result in a variation in wavelength in the range of 2:1. Further, as mentioned above, the frequency is stepped up across a range of frequencies leading to similar variations in the wavelength. Accordingly, the matching of the wavelengths may be of the same order of magnitude but need not be identical. In an ideal setup, the wavelengths may differ by up to 10% in order to ensure spatial registration between the microwave and ultrasound images, but benefits in processing may be achieved with significantly larger variations. The term "matched" should be construed accordingly.

By using an ultrasound signal with a relatively low frequency (compared to conventional ultrasonic imaging), the reconstruction is the same as for the microwave signal and the image is automatically registered and similar spatially. The low frequencies also penetrate tissue better, so will not suffer from the attenuation effects that are deleterious at higher frequencies. Further, having the same arrangement of ultrasound and microwave arrays means that at least some of the same signal processing paths (i.e. hardware) can be shared. The number of microwave antennae and ultrasonic transducers support fast data capture and processing which allows real-time 3D imaging in both cases.

Although the system has been described with reference to the imaging of a breast, it will be appreciated that it may be adapted for other areas of the body. In embodiments not forming part of the present invention, the transceiver array 6 therefore need not be hemispherical and may be contoured to conform to other body parts.

The system may employ multiple transmit and receive paths, such that data can be recorded from multiple antennas simultaneously, and may even comprise a number of transmit and receive paths corresponding to the number of antennas, such that data can be recorded from all antennas simultaneously. In an alternative topology, the switching matrix could be removed thus allowing each antenna to be connected to a transmitting/receiving device.

To avoid unnecessary duplication of effort and repetition of text in the specification, certain features are described in relation to only one or several aspects or embodiments of the invention.

The invention is not limited to the embodiments described herein, and may be modified or adapted without departing from the scope of the present invention as defined in the appended claims.

**Claims**

1. A medical imaging system (2) comprising:

   a microwave antenna array comprising a plurality of microwave antennae (16) which are spaced from one another;
   an ultrasonic transducer array comprising a plurality of ultrasonic transducers (16) which are spaced from one another and interspersed between the microwave antennae;
   wherein the plurality of microwave antennae define a transmitting antenna configured to transmit microwave signals so as to illuminate a body part of a patient and define receiving antennae configured to receive the microwave signals following interaction with the body part; and
   wherein the plurality of ultrasonic transducers define a transmitting transducer configured to transmit ultrasonic waves so as to insonate the body part of the patient and define receiving transducers configured to receive the ultrasonic waves following interaction with the body part;
   the system further comprising:

      a processor (4) configured to process the received microwave signals and the received ultrasonic waves and generate an image of the internal structure of the body part;
      wherein the microwave antennae and ultrasonic transducers are formed on a substrate (18), wherein the substrate is contoured or contourable to conform to the body part;
      wherein the substrate is hemispherical.

2. A medical imaging system as claimed in claim 1, wherein the microwave antennae and the ultrasonic transducers are wavelength-matched so as to allow similar data capture, processing, and rendering to be performed for the microwave and ultrasound modalities.

3. A medical imaging system as claimed in claim 2, wherein the wavelength of the ultrasonic waves and the wavelength of the microwave signals are substantially equal.

4. A medical imaging system as claimed in claim 3, wherein the wavelength of the ultrasonic waves and the wavelength of the microwave signals differ by up to 10%.

5. A medical imaging system as claimed in any preceding claim, wherein the microwave antennae and ultrasonic transducers emit a continuous-wave signal which is reconstructed using a phased array or delay-and-sum algorithm.

6. A medical imaging system as claimed in any preceding claim, wherein the processor is configured to generate a microwave image based on the received microwave signals and an ultrasound image based on the received ultrasonic waves, wherein the microwave and ultrasound images are spatially registered.

7. A medical imaging system as claimed in claim 6, wherein the microwave image and ultrasound image are overlaid on one another to form a compound image.

8. A medical imaging system as claimed in any preceding claim, wherein the microwave antennae and the ultrasonic transducers are arranged in corresponding formations and the microwave antenna array and the ultrasonic transducer array are offset from one another such that the ultrasonic transducers are interspersed between the microwave antennae.

9. A medical imaging system as claimed in any preceding claim,
   wherein the microwave antenna and ultrasonic transducer arrays have a plurality of configurations defining positions of the plurality of microwave antennae and ultrasonic transducers relative to the body part, wherein in each configuration the plurality of microwave antennae are located at positions which are unoccupied by microwave antennae in the other configurations and the plurality of ultrasonic transducers are located at positions which are unoccupied by ultrasonic transducers in the other configurations;
   wherein the system further comprises:

      an actuator configured to rotate the microwave antenna array and ultrasonic transducer array between the plurality of configurations so as to position the plurality of microwave antennae and the plurality of ultrasonic transducers at previously unoccupied positions; and
      wherein the processor is configured to obtain a data set for the microwave signals and ultrasonic signals produced in each of the plurality of configurations and to generate an image of the internal structure of the body part from a concatenation of the data sets.

10. A medical imaging method comprising:

illuminating a body part of a patient with microwave signals emitted by a transmitting antenna of a microwave antenna array;

receiving the microwave signals following interaction with the body part at a plurality of receiving antennae of the microwave antenna array to obtain a data set;

insonating the body part with ultrasonic waves emitted by a transmitting transducer of an ultrasonic transducer array that comprises a plurality of ultrasonic transducers which are spaced from one another and interspersed between the microwave antennae;

receiving the ultrasonic waves following interaction with the body part at a plurality of receiving transducers of the ultrasonic transducer array to obtain a data set; and

processing the received microwave signals and the received ultrasonic waves to generate an image of the internal structure of the body part;

wherein the microwave antennae and ultrasonic transducers are formed on a substrate, wherein the substrate is contoured or contourable to conform to the body part;

wherein the substrate is hemispherical.

11. A medical imaging method as claimed in claim 10, wherein the microwave antennae and the ultrasonic transducers are wavelength-matched so as to allow similar data capture, processing, and rendering to be performed for the microwave and ultrasound modalities.

12. A medical imaging method as claimed in claim 11, wherein a microwave image based on the received microwave signals and an ultrasound image based on the received ultrasonic waves is generated; and wherein the microwave and ultrasound images are spatially registered.

13. A medical imaging method as claimed in claim 12, wherein the microwave image and ultrasound image are overlaid on one another to form a compound image.

**Patentansprüche**

1. Medizinisches Bildgebungssystem (2), umfassend:

ein Mikrowellenantennenarray, das mehrere Mikrowellenantennen (16) umfasst, die voneinander beabstandet sind;

ein Ultraschallwandlerarray, das mehrere Ultraschallwandler (16) umfasst, die voneinander beabstandet und zwischen den Mikrowellenantennen eingestreut sind;

wobei die mehreren Mikrowellenantennen eine Sendeantenne definieren, die dazu ausgelegt ist, Mikrowellensignale zu übertragen, sodass ein Körperteil eines Patienten bestrahlt wird, und Empfangsantennen definieren, die dazu ausgelegt sind, die Mikrowellensignale im Anschluss an eine Interaktion mit dem Körperteil zu empfangen; und

wobei die mehreren Ultraschallwandler einen übertragenden Wandler definieren, der dazu ausgelegt ist, Ultraschallwellen zu übertragen, sodass das Körperteil des Patienten beschallt wird, und empfangende Wandler definieren, die dazu ausgelegt sind, die Ultraschallwellen im Anschluss an eine Interaktion mit dem Körperteil zu empfangen;

wobei das System ferner Folgendes umfasst:

einen Prozessor (4), der dazu ausgelegt ist, die empfangenen Mikrowellensignale und die empfangenen Ultraschallwellen zu verarbeiten und ein Bild der internen Struktur des Körperteils zu erzeugen;

wobei die Mikrowellenantennen und die Ultraschallwandler auf einem Substrat (18) ausgebildet sind, wobei das Substrat konturiert oder konturierbar ist, um dem Körperteil zu entsprechen;

wobei das Substrat halbkugelförmig ist.

2. Medizinisches Bildgebungssystem nach Anspruch 1, wobei die Mikrowellenantennen und die Ultraschallwandler wellenlängenabgeglichen sind, sodass ermöglicht wird, dass eine ähnliche Datenaufnahme, Verarbeitung und Wiedergabe für die Mikrowellen- und Ultraschallmodalitäten durchgeführt wird.

3. Medizinisches Bildgebungssystem nach Anspruch 2, wobei die Wellenlänge der Ultraschallwellen und die Wellenlänge der Mikrowellensignale im Wesentlichen gleich sind.

4. Medizinisches Bildgebungssystem nach Anspruch 3, wobei sich die Wellenlänge der Ultraschallwellen und die Wellenlänge der Mikrowellensignale um bis zu 10 % unterscheiden.

5. Medizinisches Bildgebungssystem nach einem vorangegangenen Anspruch, wobei die Mikrowellenantennen und die Ultraschallwandler ein Dauerstrichsignal emittieren, das unter Verwendung eines Phased-Array- oder Delay-and-Sum-Algorithmus rekonstruiert wird.

**6.** Medizinisches Bildgebungssystem nach einem vorangegangenen Anspruch, wobei der Prozessor dazu ausgelegt ist, ein Mikrowellenbild basierend auf den empfangenen Mikrowellensignalen und ein Ultraschallbild basierend auf den empfangenen Ultraschallwellen zu erzeugen, wobei die Mikrowellen- und Ultraschallbilder räumlich registriert sind.

**7.** Medizinisches Bildgebungssystem nach Anspruch 6, wobei das Mikrowellenbild und das Ultraschallbild einander überlagert werden, um ein zusammengesetztes Bild zu bilden.

**8.** Medizinisches Bildgebungssystem nach einem vorangegangenen Anspruch, wobei die Mikrowellenantennen und die Ultraschallwandler in entsprechenden Formationen angeordnet sind und das Mikrowellenantennenarray und das Ultraschallwandlerarray zueinander versetzt sind, sodass die Ultraschallwandler zwischen den Mikrowellenantennen eingestreut sind.

**9.** Medizinisches Bildgebungssystem nach einem vorangegangenen Anspruch,
wobei die Mikrowellenantennen- und Ultraschallwandlerarrays mehrere Konfigurationen aufweisen, die Positionen der mehreren Mikrowellenantennen und Ultraschallwandler bezüglich des Körperteils definieren, wobei, in jeder Konfiguration, sich die mehreren Mikrowellenantennen an Positionen befinden, die in den anderen Konfigurationen nicht durch Mikrowellenantennen belegt sind, und sich die mehreren Ultraschallwandler an Positionen befinden, die in den anderen Konfigurationen nicht durch Ultraschallwandler belegt sind;
wobei das System ferner Folgendes umfasst:

einen Aktor, der dazu ausgelegt ist, das Mikrowellenantennenarray und das Ultraschallwandlerarray zwischen den mehreren Konfigurationen zu drehen, sodass die mehreren Mikrowellenantennen und die mehreren Ultraschallwandler an zuvor nicht belegten Positionen positioniert werden; und
wobei der Prozessor dazu ausgelegt ist, einen Datensatz für die Mikrowellensignale und Ultraschallsignale, die in jeder der mehreren Konfigurationen erzeugt werden, zu erhalten und ein Bild der internen Struktur des Körperteils aus einer Verkettung der Datensätze zu erzeugen.

**10.** Medizinisches Bildgebungsverfahren, umfassend:

Bestrahlen eines Körperteils eines Patienten mit Mikrowellensignalen, die durch eine Sendeantenne eines Mikrowellenantennenarrays emittiert werden;
Empfangen der Mikrowellensignale im Anschluss an eine Interaktion mit dem Körperteil an mehreren Empfangsantennen des Mikrowellenantennenarrays, um einen Datensatz zu erhalten;
Beschallen des Körperteils mit Ultraschallwellen, die durch einen übertragenden Wandler eines Ultraschallwandlerarrays emittiert werden, das mehrere Ultraschallwandler umfasst, die voneinander beabstandet und zwischen den Mikrowellenantennen eingestreut sind;
Empfangen der Ultraschallwellen im Anschluss an eine Interaktion mit dem Körperteil an mehreren empfangenden Wandlern des Ultraschallwandlerarrays, um einen Datensatz zu erhalten; und
Verarbeiten der empfangenen Mikrowellensignale und der empfangenen Ultraschallwellen, um ein Bild der internen Struktur des Körperteils zu erzeugen;
wobei die Mikrowellenantennen und die Ultraschallwandler auf einem Substrat ausgebildet sind, wobei das Substrat konturiert oder konturierbar ist, um dem Körperteil zu entsprechen; wobei das Substrat halbkugelförmig ist.

**11.** Medizinisches Bildgebungsverfahren nach Anspruch 10, wobei die Mikrowellenantennen und die Ultraschallwandler wellenlängenabgeglichen sind, sodass ermöglicht wird, dass eine ähnliche Datenaufnahme, Verarbeitung und Wiedergabe für die Mikrowellen- und Ultraschallmodalitäten durchgeführt wird.

**12.** Medizinisches Bildgebungsverfahren nach Anspruch 11, wobei ein Mikrowellenbild basierend auf den empfangenen Mikrowellensignalen und ein Ultraschallbild basierend auf den empfangenen Ultraschallwellen erzeugt werden; und wobei die Mikrowellen- und Ultraschallbilder räumlich registriert sind.

**13.** Medizinisches Bildgebungsverfahren nach Anspruch 12, wobei das Mikrowellenbild und das Ultraschallbild einander überlagert werden, um ein zusammengesetztes Bild zu bilden.

**Revendications**

**1.** Système (2) d'imagerie médicale comprenant :

un réseau d'antennes micro-ondes comprenant une pluralité d'antennes micro-ondes (16) qui sont espacées les unes des autres ;
un réseau de transducteurs ultrasonores comprenant une pluralité de transducteurs ultrasonores (16) qui sont espacés les uns des autres et intercalés entre les antennes micro-ondes ;

dans lequel la pluralité d'antennes micro-ondes définit une antenne émettrice conçue pour émettre des signaux micro-ondes de façon à éclairer une partie corporelle d'un patient et définit des antennes réceptrices conçues pour recevoir les signaux micro-ondes après une interaction avec la partie corporelle ; et
dans lequel la pluralité de transducteurs ultrasonores définit un transducteur émetteur conçu pour émettre des ondes ultrasonores de façon à soumettre à l'ultrason la partie corporelle du patient et définit des transducteurs récepteurs conçus pour recevoir les ondes ultrasonores après une interaction avec la partie corporelle ; le système comprenant en outre :

un processeur (4) configuré pour traiter les signaux micro-ondes reçus et les ondes ultrasonores reçues et pour générer une image de la structure interne de la partie corporelle ;
dans lequel les antennes micro-ondes et les transducteurs ultrasonores sont formés sur un substrat (18), le substrat étant profilé ou pouvant l'être pour épouser la forme de la partie corporelle ;
dans lequel le substrat est hémisphérique.

2. Système d'imagerie médicale selon la revendication 1, dans lequel les antennes micro-ondes et les transducteurs ultrasonores correspondent en longueur d'onde de façon à permettre qu'une capture, un traitement et un rendu de données similaires soient réalisés pour les modalités micro-ondes et ultrasons.

3. Système d'imagerie médicale selon la revendication 2, dans lequel la longueur d'onde des ondes ultrasonores et la longueur d'onde des signaux micro-ondes sont sensiblement égales.

4. Système d'imagerie médicale selon la revendication 3, dans lequel la longueur d'onde des ondes ultrasonores et la longueur d'onde des signaux micro-ondes diffèrent jusqu'à 10 %.

5. Système d'imagerie médicale selon l'une quelconque des revendications précédentes, dans lequel les antennes micro-ondes et les transducteurs ultrasonores émettent un signal à onde entretenue qui est reconstruit à l'aide d'un réseau à commande de phase ou d'un algorithme par retard et sommation.

6. Système d'imagerie médicale selon l'une quelconque des revendications précédentes, dans lequel le processeur est configuré pour générer une image micro-ondes sur la base des signaux micro-ondes reçus et une image ultrasonore sur la base des ondes ultrasonores reçues, les images micro-ondes et ultrasonores étant alignées spatialement.

7. Système d'imagerie médicale selon la revendication 6, dans lequel l'image micro-ondes et l'image ultrasonore sont superposées l'une à l'autre pour former une image composée.

8. Système d'imagerie médicale selon l'une quelconque des revendications précédentes, dans lequel les antennes micro-ondes et les transducteurs ultrasonores sont agencés en formations correspondantes et le réseau d'antennes micro-ondes et le réseau de transducteurs ultrasonores sont décalés l'un de l'autre de sorte que les transducteurs ultrasonores soient intercalés entre les antennes micro-ondes.

9. Système d'imagerie médicale selon l'une quelconque des revendications précédentes,
dans lequel les réseaux d'antennes micro-ondes et de transducteurs ultrasonores ont une pluralité de configurations définissant des positions de la pluralité d'antennes micro-ondes et de transducteurs ultrasonores par rapport à la partie corporelle, dans lequel, dans chaque configuration, les multiples antennes micro-ondes sont situées en des positions qui ne sont pas occupées par des antennes micro-ondes dans les autres configurations et les multiples transducteurs ultrasonores sont situés en des positions qui ne sont pas occupées par des transducteurs ultrasonores dans les autres configurations ;
dans lequel le système comporte en outre :

un actionneur conçu pour faire tourner le réseau d'antennes micro-ondes et le réseau de transducteurs ultrasonores entre les multiples configurations de façon à positionner la pluralité d'antennes micro-ondes et la pluralité de transducteurs ultrasonores en des positions précédemment non occupées ; et
dans lequel le processeur est configuré pour obtenir un ensemble de données pour les signaux micro-ondes et les signaux ultrasonores produits dans chaque configuration de la pluralité de configurations et pour générer une image de la structure interne de la partie corporelle à partir d'une concaténation des ensembles de données.

10. Procédé d'imagerie médicale consistant à :

éclairer une partie corporelle d'un patient avec des signaux micro-ondes émis par une antenne émettrice d'un réseau d'antennes micro-ondes ;
recevoir les signaux micro-ondes après une interaction avec la partie corporelle au niveau d'une pluralité d'antennes réceptrices du réseau d'antennes micro-ondes afin d'obtenir un ensemble de données ;

soumettre la partie corporelle à des ondes ultrasonores émises par un transducteur émetteur d'un réseau de transducteurs ultrasonores qui comprend une pluralité de transducteurs ultrasonores qui sont espacés les uns des autres et intercalés entre les antennes micro-ondes ;
recevoir les ondes ultrasonores après une interaction avec la partie corporelle au niveau d'une pluralité de transducteurs récepteurs du réseau de transducteurs ultrasonores afin d'obtenir un ensemble de données ; et
traiter les signaux micro-ondes reçus et les ondes ultrasonores reçues afin de générer une image de la structure interne de la partie corporelle ;
dans lequel les antennes micro-ondes et les transducteurs ultrasonores sont formés sur un substrat, le substrat étant profilé ou pouvant l'être pour épouser la forme de la partie corporelle ;
dans lequel le substrat est hémisphérique.

11. Procédé d'imagerie médicale selon la revendication 10, dans lequel les antennes micro-ondes et les transducteurs ultrasonores correspondent en longueur d'onde de façon à permettre qu'une capture, un traitement et un rendu de données similaires soient réalisés pour les modalités micro-ondes et ultrasons.

12. Procédé d'imagerie médicale selon la revendication 11, dans lequel une image micro-ondes basée sur les signaux micro-ondes reçus et une image ultrasonore basée sur les ondes ultrasonores reçues sont générées ; et dans lequel les images micro-ondes et ultrasonores sont alignées spatialement.

13. Procédé d'imagerie médicale selon la revendication 12, dans lequel l'image micro-ondes et l'image ultrasonore sont superposées l'une à l'autre pour former une image composée.

FIG. 1

FIG. 2

Microwave Capture Phase

Ultrasonic Capture Phase

Energize microwave
antenna *m*

Detect on microwave
antennae *n = 1…N*
*n ≠ m*

*m = m + 1*

*m = N?*

No

Yes

Energize ultrasonic
transducer *m*

Detect on ultrasonic
transducers *n = 1…N*
*n ≠ m*

*m = m + 1*

*m = N?*

No

Yes

End

FIG. 3

18

16

6

FIG. 4

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102499713 B
- CN 105708492 A

- WO 2009060181 A